# EUROPÄISCHE PATENTSCHRIFT

(11) **EP 3 706 669 B1**
(45) Veröffentlichungstag und Bekanntmachung des Hinweises auf die Patenterteilung: **23.08.2023**
(21) Anmeldenummer: 18789099.1
(22) Anmeldetag: 16.10.2018
(51) Int. Cl.: A61F 2/80, A61F 2/78

(54) **ORTHOPÄDIETECHNISCHE EINRICHTUNG MIT ELEKTRISCHEM LEITER**
ORTHOPEDIC DEVICE WITH ELECTRIC CONDUCTOR
DISPOSITIF ORTHOPÉDIQUE AVEC CONDUCTEUR ÉLECTRIQUE

(30) Priorität: 10.11.2017 DE 102017126465
(43) Veröffentlichungstag der Anmeldung: 16.09.2020
(73) Patentinhaber: Ottobock SE & Co. KGaA, 37115 Duderstadt (DE)
(72) Erfinder: KOPPE, Mario, 99988 Wendehausen (DE)
(74) Vertreter: Gramm, Lins & Partner Patent- und Rechtsanwälte PartGmbB
(86) Internationale Anmeldenummer: PCT/EP2018/078232
(87) Internationale Veröffentlichungsnummer: WO 2019/091713

(56) Entgegenhaltungen:
- EP-A1- 2 737 878
- US-A1- 2009 216 339
- US-A1- 2013 046 394
- CHIA-MAN CHOU ET AL: "Deposition, characterization, and in vivo performance of parylene coating on general-purpose silicone for examining potential biocompatible surface modifications", THIN SOLID FILMS, Bd. 549, 1. Dezember 2013 (2013-12-01), Seiten 103-107, XP055532802, AMSTERDAM, NL ISSN: 0040-6090, DOI: 10.1016/j.tsf.2013.09.032

## Beschreibung

Die Erfindung betrifft eine orthopädietechnische Einrichtung mit einem Grundkörper aus einem elektrisch isolierenden Material und wenigstens einem elektrischen Leiter, der an oder in dem Grundkörper angeordnet ist, wie in den Ansprüchen definiert.

Orthopädietechnische Einrichtungen sind im Rahmen der vorliegenden Erfindung insbesondere Orthesen, Prothesen, insbesondere Prothesenschäfte und Prothesenliner, oder andere Rehabilitationsgeräte. Orthopädietechnische Einrichtungen sind heute in vielen Fällen mit Elektroden ausgerüstet, um myoelektrische Signale abzunehmen und an eine Datenverarbeitungseinrichtung weiterzuleiten oder um elektrische Stimulationssignale zur Haut und/oder einem darunterliegenden Muskel des Patienten, also des Trägers der orthopädietechnischen Einrichtung zu leiten. Dies ist insbesondere bei Prothesenlinern der Vorteil, da diese mit der Haut des Trägers unmittelbar in Kontakt kommen und daher als Träger für entsprechende Elektroden besonders geeignet sind. Selbstverständlich ist die vorliegende Erfindung jedoch nicht auf Prothesenliner beschränkt. US2013/046394 offenbart eine orthopädietechnische Einrichtung mit einem Grundkörper aus einem elektrisch isolierenden Material und wenigstens einem elektrischen Leiter, der an oder in dem Grundkörper angeordnet ist, wobei der elektrische Leiter ein elektrisch leitfähiges Elastomer aufweist.

Aus dem Stand der Technik sind eine Reihe unterschiedlicher Elektroden bekannt, die mit der Haut des Trägers der orthopädietechnischen Einrichtung in Kontakt gebracht werden können. Diese können beispielsweise auf der Innenseite der orthopädietechnischen Einrichtung, beispielsweise des Prothesenliners, also in unmittelbarem Hautkontakt, angebracht werden oder auf der Außenseite des Liners, beispielsweise an der Innenseite eines den Liner umgebenden Prothesenschaftes, positioniert sein. In diesem Fall verfügt der Liner oftmals über elektrisch leitfähige Durchleitungen, beispielsweise in Form von metallenen Nieten oder elektrisch leitfähigen Silikonbereichen, sodass die orthopädietechnische Einrichtung im Bereich der Elektroden elektrisch leitfähig gemacht wurde.

Nachteilig ist jedoch, dass in diesem Fall die Elektroden genau an der Stelle angeordnet werden müssen, an der auch die Durchleitungen in der orthopädietechnischen Einrichtung angeordnet sind und zudem direkt an der Stelle, an der sie am Träger, beispielsweise an einem Amputationsstumpf, angeordnet sein müssen. Die oftmals platzintensiven Elektroden können daher oftmals nicht an der optimalen Stelle angeordnet werden.

Aus dem Stand der Technik sind daher eine Reihe unterschiedlicher Möglichkeiten bekannt, elektrische Leiter in oder an dem Grundkörper aus dem elektrisch isolierenden Material anzuordnen. So ist es beispielsweise bekannt, Silbergewebe zu verwenden, um elektrische Signale durch den Grundkörper der orthopädietechnischen Einrichtung zu einer weiterverarbeitenden Stelle, beispielsweise an einer elektronischen Datenverarbeitungseinrichtung, zu leiten. Nachteilig bei der Verwendung von Silber ist jedoch die hohe Anfälligkeit für Korrosion und zudem die nicht ausreichende Elastizität, insbesondere, wenn ein auf diese Weise hergestellter Leiter in einer orthopädietechnischen Einrichtung angeordnet wird, die selbst aus einem elastischen Material besteht. Aus der US 2013/0046394 A1 ist eine Ausführungsform bekannt, bei der die Leiter und das Linermaterial aus einem Silikon hergestellt sein können.

Aus der DE 10 2014 106 070 A1 ist daher ein Verfahren bekannt, bei dem ein Prothesenliner mit elektrisch leitfähigen Leitern ausgerüstet wird. Dabei wird zunächst der Grundkörper aus einem elektrisch isolierenden Elastomermaterial hergestellt. Dieses wird vernetzt. Anschließend werden Strukturen in das erste ausgeformte Elastomermaterial eingebracht, die als Gussform für die elektrischen Leitungen dienen. In diese Strukturen wird anschließend ein zweites Elastomermaterial eingebracht, das elektrisch leitfähig ist und anschließend vernetzt. Auf diese Weise wird aus einem elektrisch leitfähigen Elastomermaterial ein elektrischer Leiter hergestellt, der in Vertiefungen im Grundkörper angeordnet ist.

Nachteilig ist jedoch, dass dieses Verfahren nur die Anordnung von elektrisch leitfähigen Strukturen in einer Oberfläche des Grundkörpers erlaubt und zudem aufwendig und damit zeit- und kostenintensiv ist.

Der Erfindung liegt daher die Aufgabe zugrunde, eine orthopädietechnische Einrichtung gemäß dem Oberbegriff des Anspruchs 1 so weiterzuentwickeln, dass auf besonders einfache Weise elektrische Leiter in oder an dem Grundkörper angeordnet werden können und zudem elastische Eigenschaften des Grundkörpers nicht beeinträchtigt werden.

Die Erfindung löst die gestellte Aufgabe durch eine orthopädietechnische Einrichtung gemäß dem Oberbegriff des Anspruchs 1, der sich dadurch auszeichnet, dass der wenigstens eine Leiter einen Kern aus einem elektrisch leitfähigen Elastomer und eine elektrisch isolierende Beschichtung aufweist, wobei das Elastomer ein Silikon, ein thermoplastisches Elastomer oder ein Polyurethan ist, in dem sich Graphit-, Ruß- und/oder Metallpartikel und/oder Kohlestoffnanoröhrchen und/oder leitfähige Fasern befinden und die elektrisch isolierende Beschichtung ein Parylen aufweist oder daraus besteht.

Ein solcher Leiter kann beispielsweise in Form eines Kabels oder einer Schnur vorliegen. Das Elastomer des Kerns kann beispielsweise durch Zugabe von Partikeln elektrisch leitfähig gemacht werden. Auf einen derartig geformten Kern, beispielsweise in Form der bereits genannten Schnur oder des Kabels wird eine Beschichtung aus einem elektrisch isolierenden Material aufgebracht. Dabei beeinträchtigt vorteilhafterweise die Beschichtung die elastischen und flexiblen Eigenschaften des Kerns nicht, sodass sie den elastischen und flexiblen Eigenschaften des elektrischen Leiters entsprechen. Dieser Leiter kann nun einfach in oder an dem Grundkörper der orthopädietechnischen Einrichtung angeordnet, beispielsweise in ein Elastomermaterial eines Prothesenliners eingegossen werden. Da es sich in diesem Fall sowohl beim Kern des elektrischen Leiters als auch bei dem elektrisch isolierenden Material des Grundköpers um ein Elastomer handelt, werden elastische Eigenschaften auch der orthopädietechnischen Einrichtung nicht beeinträchtigt.

Erfindungsgemäß ist ein Elastomer des Kerns ein Silikon, ein thermoplastisches Elastomer oder ein Polyurethan, in dem sich Graphit-, Ruß- und/oder Metallpartikel und/oder Kohlenstoffnanoröhrchen befinden. Alternativ oder zusätzlich können auch leitfähige Fasern verwendet werden.

Erfindungsgemäß weist die elektrisch isolierende Beschichtung ein Parylen auf oder besteht daraus. Die Verwendung von Parylen als isolierende Beschichtung hat zudem den Vorteil, dass insbesondere für den Fall, dass sowohl das elektrisch isolierende Material des Grundkörpers als auch das elektrisch leitfähige Elastomer des Leiters ein Polyurethan oder ein Silikon sind, zudem als Haftvermittler wirkt. Durch die Parylenbeschichtung wird folglich nicht nur eine elektrisch isolierende Wirkung erreicht, sondern zudem dafür gesorgt, dass zwischen dem Material des Grundkörpers der orthopädietechnischen Einrichtung und dem elektrischen Leiter eine gute Haftung besteht.

Vorzugsweise weist das Material des Grundkörpers ein Silikon, ein thermoplastisches Elastomer oder ein Polyurethan auf oder besteht daraus.

Vorteilhafterweise verfügt die orthopädietechnische Einrichtung über wenigstens eine Elektrode und/oder wenigstens einen Sensor, wobei die Elektrode und/oder der Sensor mit dem wenigstens einen Leiter elektrisch verbunden und vorzugsweise derart angeordnet ist, dass sie im an ein Körperteil angelegten Zustand der Einrichtung mit dem Körperteil in Kontakt kommt. Auf diese Weise wird ein besonders guter Kontakt zur Übermittlung elektrischer Signale und Impulse erreicht und gleichzeitig sichergestellt, dass auch bei starker mechanischer und/oder thermischer Beanspruchung der orthopädietechnischen Einrichtung eine sichere Weiterleitung der Signale gewährleistet bleibt. Der wenigstens eine Sensor weist vorzugsweise einen Drucksensor, einen Temperatur- und/oder einen Feuchtigkeitssensor, einen Pulsmess- und/oder einen Durchblutungsmesssensor (NIRS) und/oder einen Sensor zur Messung des Blutzuckerspiegels auf.

Statt die Elektroden und/oder den Sensor an der haut des Trägers der orthopädietechnischen Einrichtung anzuordnen, kann auch ein Abstand zur Haut eingehalten werden, wenn die Elektrode und/oder der Sensor kapazitiv oder induktiv arbeitet.

Vorzugsweise verfügt der wenigsten eine Leiter über eine Abschirmung aus einem elektrisch leitfähigen Elastomer, die auf der dem Kern abgewandten Seite der isolierenden Beschichtung angeordnet ist. Der elektrische Leiter ist folglich von radial innen nach radial außen dreischichtig aufgebaut und verfügt zunächst innen über den Kern aus elektrisch leitfähigem Material, vorzugsweise einem Elastomer, das von der elektrisch isolierenden Beschichtung abgedeckt wird, auf deren Außenseite sich die Abschirmung befindet. Auf diese Weise ist es möglich, eine Art einen Koaxialkabels abgeschirmten elektrischen Leiter herzustellen, der die elastischen und flexiblen Eigenschaften insbesondere eines Prothesenliners nicht beeinträchtigt.

Bevorzugt weist die Abschirmung das gleiche Material wie der Kern des elektrischen Leiters auf oder besteht daraus.

Mit Hilfe der beigefügten Zeichnung wird nachfolgend ein Ausführungsbeispiel der vorliegenden Erfindung näher erläutert.

Es zeigen
- Figur 1 -: schematische Schnittdarstellungen durch einen Leiter für eine orthopädietechnische Einrichtung gemäß einem ersten Ausführungsbeispiel der vorliegenden Erfindung und
- Figuren 2 bis 6 -: verschiedene orthopädietechnische Einrichtungen gemäß weiteren Ausführungsbeispielen der vorliegenden Erfindung.

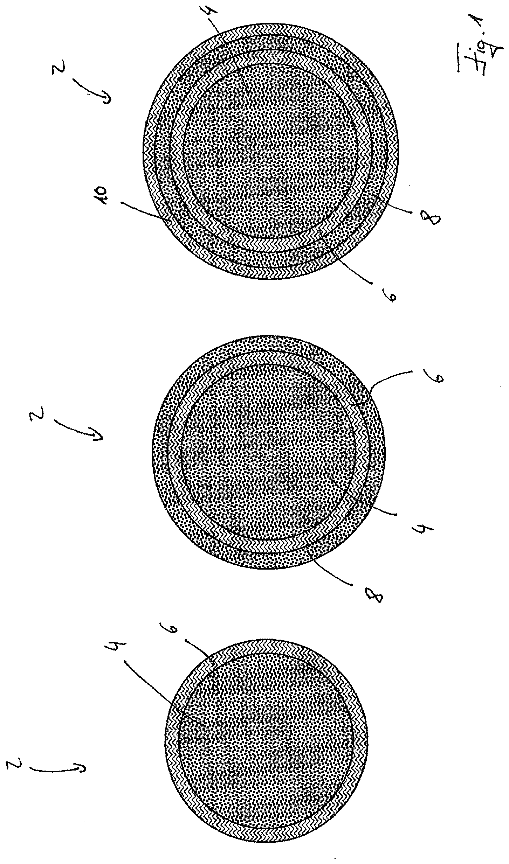

Figur 1 zeigt in der linken Darstellung einen Schnitt durch einen elektrischen Leiter 2 für eine orthopädietechnische Einrichtung gemäß einem ersten Ausführungsbeispiel der vorliegenden Erfindung. Der elektrische Leiter 2 verfügt über einen Kern 4 aus einem elektrisch leitfähigen Elastomer, beispielsweise einem mit Graphit-Partikeln versetzten Silikon. Der Kern 4 ist umgeben von einer Beschichtung 6, die aus einem elektrisch isolierenden Material, beispielsweise Parylen besteht.

In der mittleren Darstellung der Figur 1 ist ein elektrischer Leiter 2 dargestellt, bei dem der Kern 4 mit der ihn umgebenden Beschichtung 6 von einer Abschirmung 8 umgeben ist, die ebenfalls aus einem elektrisch leitfähigen Elastomer besteht und auf der dem Kern 4 abgewandten Seite der isolierenden Beschichtung 6 angeordnet ist. Auf diese Weise lässt sich ein elektrischer Leiter 2 herstellen, der die Eigenschaften eines Koaxialkabels mit einer elektrischen Abschirmung 8 aufweist.

In der rechten Darstellung der Figur 1 ist ein elektrischer Leiter 2 dargestellt, der wie in der mittleren Darstellung eine Abschirmung 8 aufweist, die jedoch nun von einer zweiten Beschichtung 10 umgeben ist. Bei der zweiten Beschichtung 10 handelt es sich wie bei der Beschichtung 6 um eine elektrisch isolierende Beschichtung, die vorteilhafterweise aus dem gleichen Material wie die Beschichtung 6 besteht.

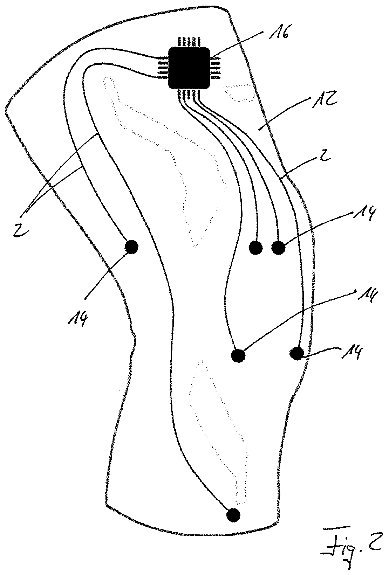

Figur 2 zeigt eine orthopädietechnische Einrichtung in Form einer Kniebandage. Sie verfügt über einen Grundkörper 12, der beispielsweise aus einem elastischen Textil hergestellt sein kann und über Verdickungen, Pelotten oder eingeschobene oder eingearbeitete Polsterelemente verfügen kann. Im gezeigten Ausführungsbeispiel sind in dem Grundkörper 12 sechs Elektroden 14 eingearbeitet, die jeweils mit einem elektrischen Leiter 2 verbunden sind. Die elektrischen Leiter 2 verbinden die Elektroden 14 mit einer elektrischen Steuerung 16, die in Figur 2 schematisch dargestellt ist. Die elektrische Steuerung 16 ist eingerichtet, elektrische Signale, die von den Elektroden 14 über die elektrischen Leiter 2 an die elektrische Steuerung 16 gesendet werden, weiter zu verarbeiten und gegebenenfalls an eine elektronische Datenverarbeitungseinrichtung zu übermitteln. Dies kann beispielsweise über in Figur 2 nicht dargestellte Kabel geschehen, was insbesondere dann von Vorteil ist, wenn die elektronische Datenverarbeitungseinrichtung in der elektrischen Steuerung 14 oder zumindest am Grundkörper 12 der orthopädietechnischen Einrichtung angeordnet ist. So kann es beispielsweise sinnvoll sein, die von den Elektroden 14 übermittelten elektrischen Signale in Form von elektronischen Daten in der elektrischen Steuerung 16 so weiterzuentwickeln, dass sie in einem elektronischen Datenspeicher, der vorteilhafterweise Teil der orthopädietechnischen Einrichtung ist, hinterlegt werden können, bis sie ausgelesen und ausgewertet werden können. Alternativ oder zusätzlich dazu ist es von Vorteil, eine drahtlose Übermittlung der elektronischen Daten von der elektrischen Steuerung 16 auf eine elektronische Datenverarbeitungseinrichtung zu ermöglichen.

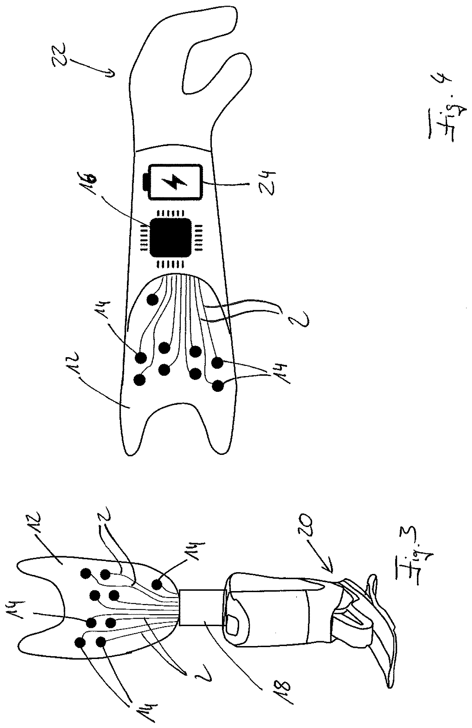

Figur 3 zeigt eine orthopädietechnische Einrichtung gemäß einem weiteren Ausführungsbeispiel der vorliegenden Erfindung in Form einer Unterschenkelprothese. Der Grundkörper 12 ist in Form eines Prothesenschaftes ausgebildet, an dem die Elektroden 14 angeordnet und über elektrische Leiter 2 mit einer elektrischen Steuerung verbunden sind, die nicht dargestellt ist. Am Grundkörper 12 befindet sich ein Unterschenkelelement 18 sowie ein künstlicher Fuß 20. Über die Elektroden 14 können beispielsweise myoelektrische Signale von einem Amputationsstumpf abgenommen werden, der in dem Grundkörper 12 angeordnet wird. Diese myoelektrischen Signale werden über die elektrischen Leiter 2 zur elektrischen Steuerung geleitet und zur Steuerung des künstlichen Fußes 18 verwendet.

Figur 4 zeigt eine weitere Ausführungsform einer orthopädietechnischen Einrichtung gemäß einem Ausführungsbeispiel der vorliegenden Erfindung, die ebenfalls als Prothese, nun jedoch als Handprothese und Unterarmprothese ausgebildet ist. Auch hier bildet der Grundkörper 12 einen Prothesenschaft, an dem die Elektroden 14 angeordnet und über elektrische Leiter 2 mit der elektrischen Steuerung 16 verbunden sind. Die elektrische Steuerung 16 ist eingerichtet, aus den von den Elektroden 14 abgenommenen elektrischen Signalen Steuersignale für eine Prothesenhand 22 zu generieren und so Funktionen der Prothesenhand 22 zu steuern. Die in Figur 4 gezeigte orthopädietechnische Einrichtung verfügt zudem über eine Stromversorgung 24, durch die die elektrische Steuerung 16 mit elektrischem Strom versorgbar ist.

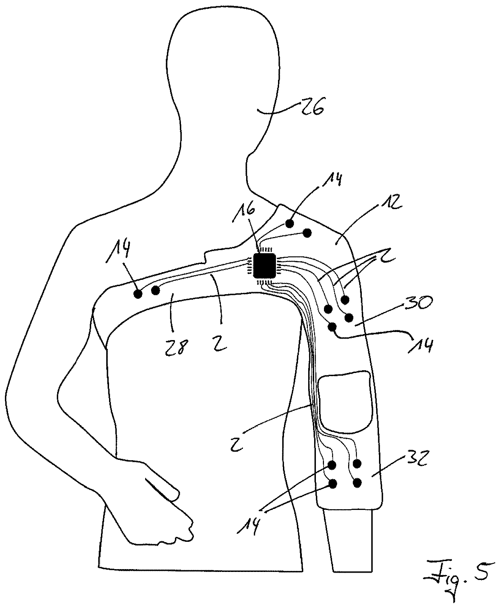

Figur 5 zeigt eine Ausführungsform einer orthopädietechnischen Einrichtung, die als Schulterorthese ausgebildet ist. Der Grundkörper 12 erstreckt sich über den Schulterbereich des Trägers 26 entlang des Arms des Trägers 26 bis über den Ellbogen hinaus. Der Grundkörper 12 verfügt über einen Befestigungsgurt 28, der um den Rumpf des Trägers 24 herumgeführt wird. Sowohl an dem Befestigungsgurt 28, der Teil des Grundkörpers 12 ist, als auch an anderen Teilen des Grundkörpers 12, beispielsweise an einem Oberarmelement 30 und einem Unterarmelement 32 sind Elektroden 14 angeordnet, die durch elektrische Leiter 2 mit der elektrischen Steuerung 16 verbunden sind.

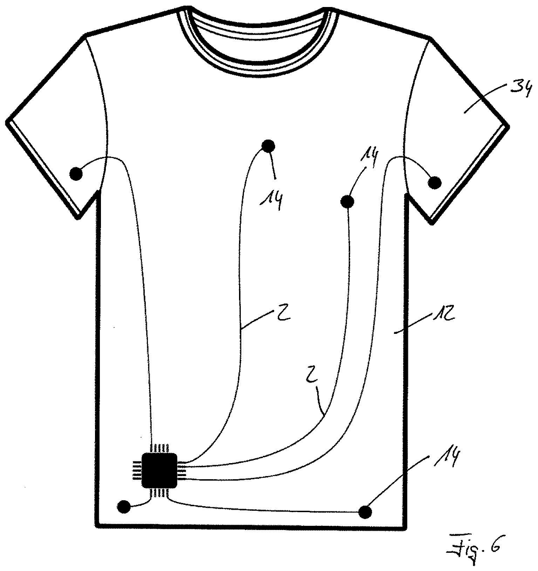

Figur 6 zeigt eine orthopädietechnische Einrichtung in Form eines T-Shirts, wobei der Grundkörper 12 das T-Shirt selbst bildet. Sowohl an Ärmeln 34 als auch am Rest des Grundkörpers 12 sind Elektroden 14 angeordnet, die über elektrische Leiter 2 mit der elektrischen Steuerung 16 verbunden sind. Die Schwierigkeit bei dieser Ausgestaltung der orthopädietechnischen Einrichtung besteht darin, einen möglichst guten Kontakt zwischen den Elektroden 14 und dem Hautbereich des Trägers 24 aufzubauen. Dies kann beispielsweise über den Schnitt des T-Shirts, ein elastisches Material oder eingearbeitete Gurte geschehen.

### Bezugszeichenliste

- 2: elektrischer Leiter
- 4: Kern
- 6: Beschichtung
- 8: Abschirmung
- 10: zweite Beschichtung
- 12: Grundkörper
- 14: Elektrode
- 16: elektrische Steuerung
- 18: Unterschenkelelement
- 20: künstlicher Fuß
- 22: Prothesenhand
- 24: Stromversorgung
- 26: Träger
- 28: Befestigungsgurt
- 30: Oberarmelement
- 32: Unterarmelement
- 34: Ärmel

## Patentansprüche

1. Orthopädietechnische Einrichtung mit
einem Grundkörper (12) aus einem elektrisch isolierenden Material und
wenigstens einem elektrischen Leiter (2),
der an oder in dem Grundkörper (12) angeordnet ist, wobei der elektrische Leiter ein elektrisch leitfähiges Elastomer aufweist,
**dadurch gekennzeichnet, dass**
der wenigstens eine Leiter (2) einen Kern (4) aus dem elektrisch leitfähigen
Elastomer und eine elektrisch isolierende Beschichtung (6) aufweist, wobei das Elastomer ein Silikon, ein thermoplastisches Elastomer oder ein Polyurethan ist, in dem sich Graphit-, Ruß- und/oder Metallpartikel und/oder Kohlestoffnanoröhrchen und/oder leitfähige Fasern befinden und die elektrisch isolierende Beschichtung (6) ein Parylen aufweist oder daraus besteht.

2. Orthopädietechnische Einrichtung nach einem der vorstehenden Ansprüche, **dadurch gekennzeichnet, dass** das Material des Grundkörpers (12) Silikon, ein thermoplastisches Elastomer oder ein Polyurethan aufweist oder daraus besteht.

3. Orthopädietechnische Einrichtung nach einem der vorstehenden Ansprüche, **dadurch gekennzeichnet, dass** die Einrichtung wenigstens eine Elektrode (14) und/oder wenigstens einen Sensor aufweist, wobei die Elektrode (14) und/oder der Sensor mit dem wenigstens einen Leiter (2) elektrisch verbunden und vorzugsweise derart angeordnet ist, dass sie im an ein Körperteil angelegten Zustand der Einrichtung mit dem Körperteil in Kontakt kommt.

4. Orthopädietechnische Einrichtung nach einem der vorstehenden Ansprüche, **dadurch gekennzeichnet, dass** der wenigstens eine Leiter (2) eine Abschirmung (8) aus einem elektrisch leitfähigen elastischen Material, vorzugsweise einem Elastomer aufweist, die auf der dem Kern (4) abgewandten Seite der isolierenden Beschichtung (6) angeordnet ist.

5. Orthopädietechnische Einrichtung nach Anspruch 4, **dadurch gekennzeichnet, dass** die Abschirmung (8) das gleiche Material wie der Kern (4) aufweist oder daraus besteht.

## Claims

1. An orthopaedic device with
a base body (12) made of an electrically insulating material and
at least one electric conductor (2),
which is arranged on or in the base body (12), wherein the electric conductor comprises an electrically conductive elastomer,
**characterized in that**
the at least one conductor (2) comprises a core (4) made of the electrically conductive elastomer and an electrically insulating coating (6), wherein the elastomer is a silicone, a thermoplastic elastomer or a polyurethane, in which graphite, soot and/or metal particles and/or carbon nanotubes and/or conductive fibers are found and the electrically insulating coating (6) comprises or is made of a parylene.

2. The orthopaedic device according to one of the above claims, **characterized in that** the material of the base body (12) comprises or is made of silicone, a thermoplastic elastomer or a polyurethane.

3. The orthopaedic device according to one of the above claims, **characterized in that** the device comprises at least one electrode (14) and/or at least one sensor, wherein the electrode (14) and/or the sensor is electrically connected to the at least one conductor (2) and is preferably arranged such that, when the device is mounted on a body part, it comes into contact with that body part.

4. The orthopaedic device according to one of the above claims, **characterized in that** the at least one conductor (2) comprises a shielding (8) made of an electrically conductive elastic material, preferably an elastomer, which is arranged on the side of the insulating coating (6) that faces away from the core (4).

5. The orthopaedic device according to claim 4, **characterized in that** the shielding (8) comprises or is made of the same material as the core (4).

## Revendications

1. Dispositif orthopédique, comprenant
un corps de base (12) en un matériau électriquement isolant et au moins un conducteur électrique (2) disposé sur ou dans le corps de base (12), le conducteur électrique comprenant un élastomère électriquement conducteur,
**caractérisé en ce que**
ledit au moins un conducteur (2) présente une âme (4) en ledit élastomère électriquement conducteur et un revêtement électriquement isolant (6), l'élastomère étant un silicone, un élastomère thermoplastique ou un polyuréthane dans lequel se trouvent des particules de graphite, de noir de carbone et/ou de métal et/ou des nanotubes de carbone et/ou des fibres conductrices, et le revêtement électriquement isolant (6) contient ou est constitué d'un parylène.

2. Dispositif orthopédique selon la revendication précédente,
**caractérisé en ce que** le matériau du corps de base (12) contient ou est constitué de silicone, d'un élastomère thermoplastique ou d'un polyuréthane.

3. Dispositif orthopédique selon l'une des revendications précédentes, **caractérisé en ce que** le dispositif comporte au moins une électrode (14) et/ou au moins un capteur, l'électrode (14) et/ou le capteur étant relié(e) électriquement audit au moins un conducteur (2) et étant de préférence disposé(e) de manière à venir en contact avec une partie du corps lorsque le dispositif est appliqué sur la partie du corps.

4. Dispositif orthopédique selon l'une des revendications précédentes, **caractérisé en ce que** ledit au moins un conducteur (2) présente un blindage (8) en un matériau élastique électriquement conducteur, de préférence un élastomère, qui est disposé sur le côté du revêtement isolant (6) détourné de l'âme (4).

5. Dispositif orthopédique selon la revendication 4,
**caractérisé en ce que** le blindage (8) comprend ou est constitué du même matériau que l'âme (4).
